# EUROPEAN PATENT SPECIFICATION

(11) **EP 3 250 220 B1**
(45) Date of publication and mention of the grant of the patent: **11.03.2020**
(21) Application number: 16701645.0
(22) Date of filing: 27.01.2016
(51) Int. Cl.: A61K 38/17, A61P 35/00

(54) **AGONISTS OF THE NOGO-A S1PR PATHWAY FOR THE TREATMENT OF GLIOBLASTOMA**
AGONISTEN DES NOGO-A S1PR-PFADS ZUR BEHANDLUNG VON GLIOBLASTOMEN
AGONISTES DE LA VOIE S1PR NOGO-A POUR LE TRAITEMENT DU GLIOBLASTOME

(30) Priority: 27.01.2015 EP 15152762
(43) Date of publication of application: 06.12.2017
(73) Proprietor: Universität Zürich, 8006 Zürich (CH)
(72) Inventor: WÄLCHLI,Thomas, 8006 Zürich (CH); HOERSTRUP, Simon, 8032 Zürich (CH); FREI, Karl, 8645 Jona (CH); SCHWAB, Martin, 8057 Zürich (CH); WACKER, Andrin, 8610 Uster (CH)
(74) Representative: Schulz Junghans Patentanwälte PartGmbB
(86) International application number: PCT/EP2016/051614
(87) International publication number: WO 2016/120290

(56) References cited:
- WO-A2-00/31235
- HONG LIAO ET AL: "Nogo-66 and myelin-associated glycoprotein (MAG) inhibit the adhesion and migration of Nogo-66 receptor expressing human glioma cells", JOURNAL OF NEUROCHEMISTRY, vol. 90, no. 5, 1 September 2004 (2004-09-01), pages 1156-1162, XP055185209, ISSN: 0022-3042, DOI: 10.1111/j.1471-4159.2004.02573.x
- A. ESTRADA-BERNAL ET AL: "Induction of brain tumor stem cell apoptosis by FTY720: a potential therapeutic agent for glioblastoma", NEURO-ONCOLOGY, vol. 14, no. 4, 20 February 2012 (2012-02-20), pages 405-415, XP055185571, ISSN: 1522-8517, DOI: 10.1093/neuonc/nos005
- LI ZHANG ET AL: "FTY720 reduces migration and invasion of human glioblastoma cell lines via inhibiting the PI3K/AKT/mTOR/p70S6K signaling pathway", TUMOR BIOLOGY, vol. 35, no. 11, 30 July 2014 (2014-07-30) , pages 10707-10714, XP055185560, ISSN: 1010-4283, DOI: 10.1007/s13277-014-2386-y
- T. WALCHLI ET AL: "Nogo-A is a negative regulator of CNS angiogenesis", PROCEEDINGS OF THE NATIONAL ACADEMY OF SCIENCES, vol. 110, no. 21, 21 May 2013 (2013-05-21) , pages E1943-E1952, XP55185229, ISSN: 0027-8424, DOI: 10.1073/pnas.1216203110

## Description

Brain tumors and especially glioblastoma are deadly cancers characterized by uncontrollable growth leading to death within 15 months in average (for glioblastoma). One hallmark of brain tumor growth is angiogenesis, the growth of new blood vessels. In principle, the inhibition of exuberant angiogenesis in brain tumors/glioblastoma can inhibit/slow down brain tumor growth. However, any current strategy to slow down brain tumor/glioblastoma growth has not resulted in the desired improvement of patient survival.

In the adult central nervous system (CNS), Nogo-A is a myelin-associated protein mostly synthesized by oligodendrocytes. Nogo-A was identified as a major inhibitory molecule for axonal regeneration in the CNS.

Nogo-A exerts its inhibitory effects via two domains: Nogo-66 (human aa 1055-1120), which is present in all three major Nogo isoforms (Nogo-A, Nogo-B and Nogo-C) and Nogo-A Delta 20 (human aa 567-748), which is specific to Nogo-A. It is also know in the art that Nogo-A Delta 20, but not Nogo-66 is involved in the regulation of angiogenesis during mouse brain development (Wälchli et al., PNAS 2013;110, E1943-52).

Nogo-66 signals by means of a multimeric receptor complex composed of the Nogo-66 receptor (NgR1), the adaptor element Lingo1, the effector part p75/Troy and the co-receptor PirB. The stimulation of NgR - p75/Troy - Lingo1 by Nogo-A/Nogo-66 leads to the activation of the intracellular GTPase RhoA. RhoA is thought to be a key mediator of Nogo-A causing actin depolymerization and thereby neurite retraction. The receptor for the Nogo-A specific inhibitory domain Nogo-A Delta 20 was only recently identified as the sphingolipid receptor sphingosin-1 phosphate (S1P) receptor 2 (S1PR2).

Nogo-A is an important negative regulator of postnatal CNS angiogenesis. Nogo-A is expressed in neurons in immediate vicinity of CNS endothelial tip cell filopodia, suggesting a neurovascular cellular mechanism. Genetic deletion or antibody-mediated neutralization of Nogo-A led to increased CNS tissue vessel density and increased number of CNS endothelial tip cells. In pathological conditions, very little is known about a possible influence of Nogo-A on angiogenesis.

Despite multimodal treatment by surgery followed by radio- and chemotherapy, commonly temozolomide, the median survival of glioblastoma patients is only around 1 year. Given the lack of therapeutic success in the treatment of brain tumors as well as the important role for Nogo-A signaling as a negative regulatory pathway of angiogenesis, functional modulation of Nogo-A and its receptors in brain tumor angiogenesis harbors great potential.

The present invention is based on the surprising, unexpected finding that Nogo-A and its receptors interact to negatively regulate the spreading and migration of human glioblastoma-derived endothelial cells.

The problem underlying the present invention is to provide a safe and efficacious treatment of glioblastoma. This problem is solved by the subject matter of the independent claims.

### Terms and definitions

Gene ID numbers in this document refer to entries in the Gene data base of the United States National Center for Biotechnology Information.

Glioblastoma growth in the sense of the instant invention means exuberant, uncontrollable growth of brain tumors finally leading to damage of the nervous system, various neurological symptoms including movement paresis, speech problems, visual problems, personality changes etc. and for most patients to death within 15 months in average.

According to a first aspect of the invention, an isolated polypeptide comprising, or essentially consisting of, an extracellular domain of Nogo-A delta 20 (identified by SEQ ID NO 001 and SEQ ID NO 002) or a functionally equivalent homologue thereof is provided for the use in a method for the treatment of glioblastoma.

In certain embodiments the extracellular domain of Nogo is functionally equivalent to any one of SEQ ID NOs 001 or 002 and has • 70%, • 80%, • 85%, • 90%, • 92%, • 93%, • 94%, • 95%, • 96%, • 97% or • 98%, or 100%, sequence identity compared to any one of SEQ ID NOs 001 or 002.

Without wishing to be bound by theory, the inventors hypothesize that the interaction of the extracellular domains of Nogo with their respective receptor(s) triggers pathways involved in the regulation of angiogenesis. Therefore a compound different to the extracellular domains of Nogo, but similar in structure (or receptor effect) to Nogo-A may interact with the same receptors as Nogo-A delta 20 and/or Nogo-66. Binding of such a compound to the receptor activates the signalling pathway downstream of the receptor similar to Nogo-A extracellular domains.

According to a second aspect of the invention, a pharmaceutical composition is provided for use in a method for the treatment of glioblastoma. The pharmaceutical composition comprises a polypeptide comprising, or essentially consisting of, an extracellular domain of Nogo, specifically Nogo-A delta 20.

In certain embodiments, the extracellular domain is functionally equivalent to any one of SEQ ID NOs 001 or 002 and has • 70%, • 80%, • 85%, • 90%, • 92%, • 93%, • 94%, • 95%, • 96%, • 97% or • 98% sequence identity compared to SEQ ID NOs 001 or 002. In certain embodiments, the extracellular domain has the indicated sequence identities compared to SEQ ID NOs 001 or 002 and is functionally equivalent with Nogo delta 20 with respect to Nogo delta 20's interaction with the S1PR2 receptor.

In certain embodiments the pharmaceutical composition is applied by intravenous or intrathecal or intraventricular administration.

According to another aspect of the invention, an agonist capable of binding to a member of the S1PR receptor group comprised of S1PR1 (Gene ID: 1901), S1PR2 (Gene ID: 9294), S1PR3 (Gene ID: 1903) and S1PR5 (Gene ID: 53637) with a dissociation constant of 10⁻⁸ mol/l or smaller is provided for treating/inhibiting glioblastoma growth. In certain embodiments the agonist binds to S1PR2 and S1PR1. In certain embodiments the agonist binds to S1PR2 and S1PR3. In certain embodiments the agonist binds to S1PR2 and S1PR5. In certain embodiments the agonist binds S1PR2.

In certain embodiments, the S1PR receptor agonist is Nogo-A Delta 20, which is provided for use in a method for treating glioblastoma, particularly glioblastoma multiforme. Nogo-A Delta 20 is a peptide activator for S1PR2 but also binds other S1P receptors.

According to another aspect of the invention, an agonist (e.g. Nogo-66) capable of binding to the Nogo-66 receptor complex comprised of NgR1 (Gene ID: 65078), Lingo1 (Gene ID: 84894), p75 (Gene ID: 7133), TROY (Gene ID: 55504), and PirB (Gene ID: 11025) with a dissociation constant of 10⁻⁸ mol/l or smaller is provided for treating glioblastoma growth by inhibition of glioblastoma angiogenesis.

In certain embodiments, Nogo-66 (SEQ ID NOs 003 and 004) is provided for use in a method for treating glioblastoma, particularly glioblastoma multiforme by inhibition of glioblastoma angiogenesis.

In certain embodiments, the extracellular domain of Nogo for use in a method for the treatment of glioblastoma by inhibition of glioblastoma angiogenesis has • 70%, • 80%, • 85%, • 90%, • 92%, • 93%, • 94%, • 95%, • 96%, • 97% or • 98% sequence identity compared to any one of SEQ ID NO 003 or SEQ ID NO 004.

The invention is further illustrated by the following examples and figures, from which further embodiments and advantages can be drawn. These examples are meant to illustrate the invention but not to limit its scope.

### Short description of the figures

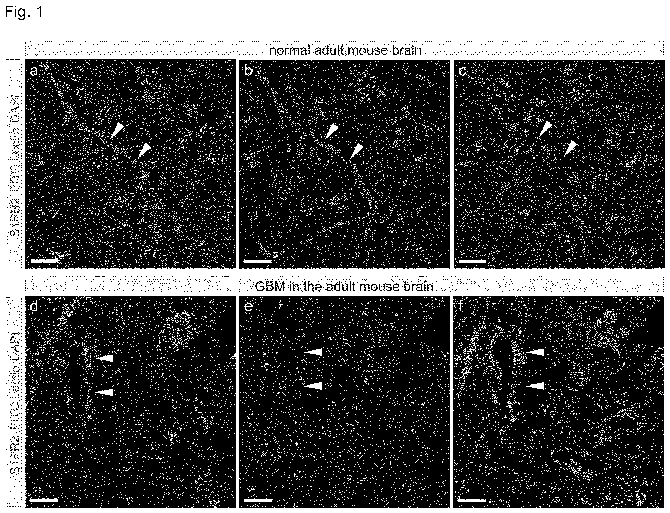

Fig. 1 shows expression of S1PR2 in a mouse model of glioblastoma. Coronal brain sections (40 µm) of glioblastoma-bearing mice (15 days post-injection, 15D-PI) that were perfused with FITC Lectin (green) to visualize blood vessels and stained for S1PR2 (red), and the general nuclear marker DAPI (blue). (a-c) Blood vessels (green, arrows) in the normal brain adjacent to the glioblastoma express S1PR2 (red). (d-f) Within the tumor/glioblastoma/glioma, S1PR2 on blood vessels (green, arrows) is upregulated. Scale bars: 20 • m in a-f

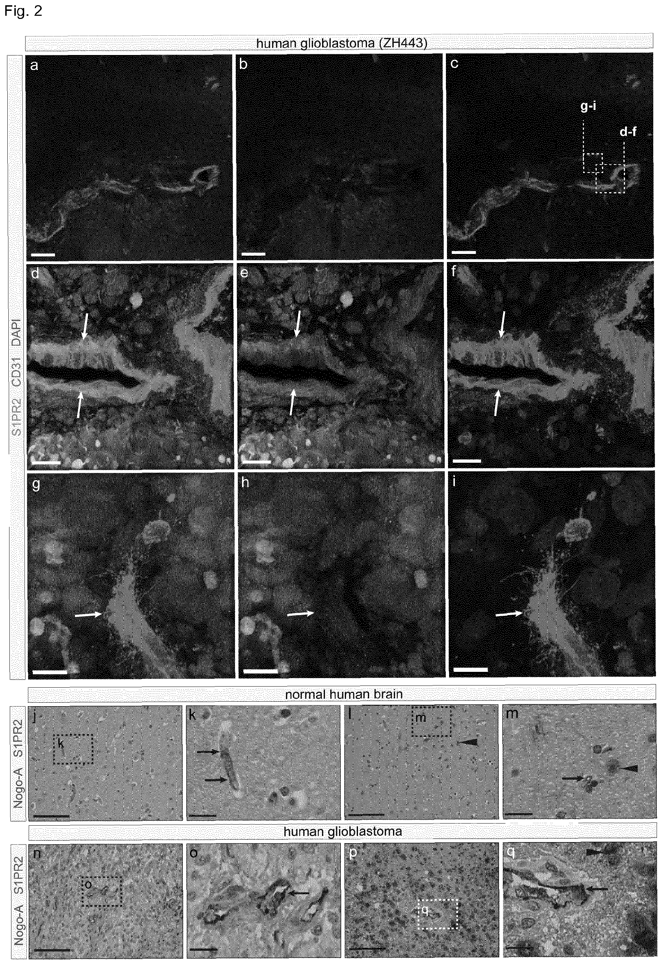

Fig. 2 shows expression of Nogo-A and S1PR2 in human glioblastoma tissue. Sections (80 µm) of human glioblastoma tissue were stained for S1PR2 (green), the vascular endothelial marker CD31 (red) and the general nuclear marker DAPI (blue). (a-c) Blood vessels (red) within the human glioblastoma tissue express S1PR2 (green). The boxed areas are enlarged in d-f and in g-i. (d-i) S1PR2 is expressed on an established blood vessel (red, arrows) (d-f) as well as on endothelial tip cells (red, arrows) (g-i) within the glioblastoma. (j-q) Tissue microarrays (10 µm) of human glioblastoma and normal human brain samples were stained for Nogo-A (red) and for S1PR2 (brown). S1PR2 (brown) is expressed on endothelial cells of normal human brain as well as on tumor endothelial cells of human glioblastoma tissues (arrows). Nogo-A (red) shows a highly variable expression in human glioblastoma ranging from virtually absent (n,o) to strong expression (p,q). Nogo-A expression on tumor endothelial cells was almost never observed (n-q). Scale bars: 100µm in a,b,c,j,l,n,p; 20µm in k,m,o,q; 10 µm in d-i

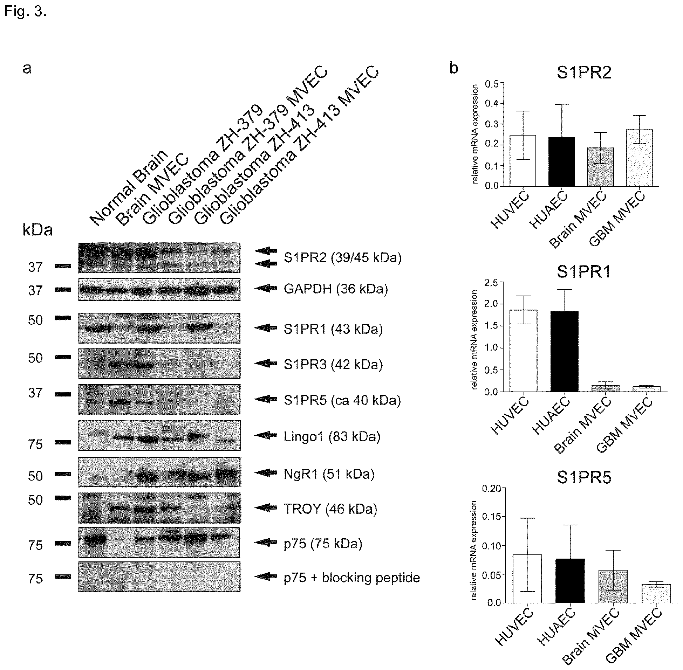

Fig. 3 shows expression of the Nogo-A receptors in human glioblastoma tissue and in isolated human glioblastoma endothelial cells. (a) Western blots using antibodies against the different proteins of the receptors for Nogo-A Delta 20 and for Nogo-66. S1P receptors (S1PR1,2,3,5) were detected in human glioblastoma extracts (Glioblastoma ZH-379/413) and in isolated human glioblastoma endothelial cells (Glioblastoma ZH-379/413 MVEC). Protein members of the Nogo-66 receptor complex, namely NgR1, Lingo1, TROY, p75 and PirB were detected in human glioblastoma extracts (Glioblastoma ZH-379/413 and in isolated human glioblastoma endothelial cells (Glioblastoma ZH-379/413 MVEC). (b) Expression of S1PR2, S1PR1, and S1PR5 was also detected in normal brain MVEC and glioblastoma MVEC (GBM MVEC) using quantitative RT-PCR. The expression was comparable to HUVEC and HUAEC except for S1PR1, which was stronger in HUVEC and HUAEC.

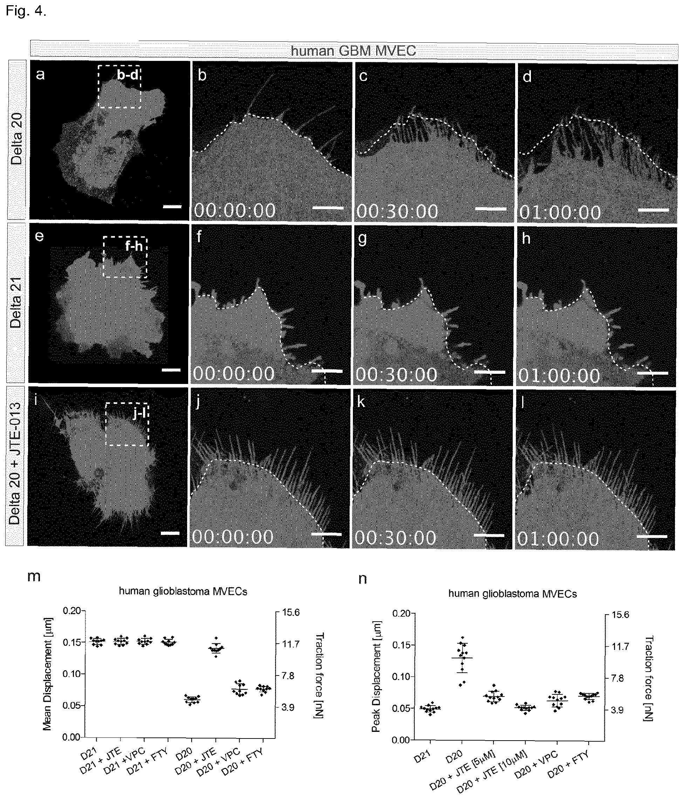

Fig. 4 shows that Nogo-A and S1PR2 interact to inhibit the spreading and filopodia and lamellipodia extension of isolated human glioblastoma endothelial cells using the pharmacological inhibitor of S1PR2 - JTE-013 - as well as other pharmacological inhibitors of the S1PR family receptors (VPC 23019 and FTY720). (a-d) Time course showing the retraction of isolated human glioblastoma microvascular endothelial cell (GBM MVECs) lamellipodia and filopodia over 30 min to 1 h, induced by Nogo-A Delta 20 at a final concentration of 1 • M. (e-h) Nogo-A Delta 21-treated GBM MVEC showed only minor shape changes. (i-l) Soluble Nogo-A Delta 20 (1 • M)-induced retraction of GBM MVEC lamellipodia and filopodia was counterbalanced by pre-incubation of GBM MVECs with JTE-013 (10 • M), a pharmacological inhibitor of S1PR2. The boxed areas are enlarged on the right side. (m) Mean traction forces and pillar displacements induced by adhered GBM MVECs on nanopillars were reduced on Nogo-A Delta 20-coated nanopillars but not on Nogo-A Delta 21-coated nanopillars. Nogo-A Delta 20 coating induced mean traction forces on the underlying nanopillars of -12 nN. Pre-incubation of GBM MVECs with 10 •M of the pharmacological S1PR2 inhibitor JTE-013 decreased the mean traction forces induced by Nogo-A Delta 20 coating onto the nanopillars to -4.1 nN. However, pre-incubation of GBM MVECs with 10 • M of the pharmacological inhibitor of S1PR1 and S1PR3, VPC 23019 or with the pharmacological inhibitor of S1PR1,3,4, or 5, FTY720 did not decrease the mean traction forces induced by Nogo-A Delta 20 coating onto the nanopillars, (n) Peak traction forces exerted by GBM MVEC filopodia onto nanopillars. Nogo-A Delta 20 (1 • M) treated GBM MVEC filopodia induced peak traction forces on the underlying nanopillars of -10 nN, while treatment with the control peptide Nogo-A Delta 21 induced peak traction forces of only -4 nN. Pre-incubation of GBM MVECs with 10 • M of the pharmacological S1PR2 inhibitor JTE-013 decreased the average peak traction forces induced by Nogo-A Delta 20 (1 • M) treated GBM MVEC filopodia onto the nanopillars to -4.5 nN, almost down to the levels of Nogo-A Delta 21 (1 • M)-treated GBM MVEC filopodia (∼4 nN). Interestingly, pre-incubation of GBM MVECs with 10 • M of the pharmacological inhibitor of S1PR1 and S1PR3, VPC 29013 or with the pharmacological inhibitor of S1PR1,3,4, or 5, FTY720 did also decrease the average peak traction forces induced by Nogo-A Delta 20 (1 • M) treated GBM MVEC filopodia onto the nanopillars, to levels close to Nogo-A Delta 21.

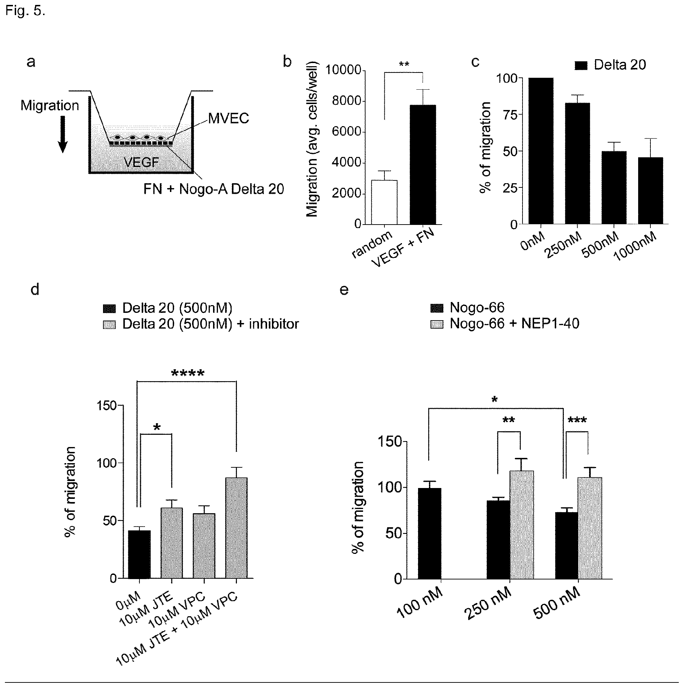

Fig. 5 shows that Nogo-A and S1PR2 as well as Nogo-66 and the Nogo-66 receptor complex interact to inhibit the migration of isolated human glioblastoma endothelial cells using the pharmacological inhibitor of S1PR2, JTE-013, other pharmacological inhibitors of the S1PR family receptors (VPC 23019 and FTY720) as well as the competitive antagonist of Nogo-66, NEP1-40, preventing the activation of the Nogo-66 receptor complex. (a,b) Human GBM MVECs show induced migration upon stimulation with a combination of VEGF-A and Fibronectin (VEGF+FN) (schematic in (a) and data in (b)). (c) VEGF+FN-induced migration of human GBM MVECs is dose-dependently inhibited by the addition of Nogo-A Delta 20. (d) Pre-incubation of GBM MVECs with 10 • M of the pharmacological S1PR2 inhibitor JTE-013 significantly increases the migration potential despite Nogo-A Delta 20 exposure. Although VPC 23019, a pharmacological blocker for S1PR1 and S1PR3, has no effect on its own, combined pre-incubation with JTE-013 lead to a strong reduction of the inhibitor effect of Nogo-A Delta 20 on GBM MVEC migration. (e) VEGF+FN-induced migration of human GBM MVECs is dose-dependently inhibited by the addition of Nogo-66. Pre-incubation with NEP1-40, a competitive antagonist of Nogo-66, significantly increases the migration potential GBM MVEC upon exposure to Nogo-66 coating.

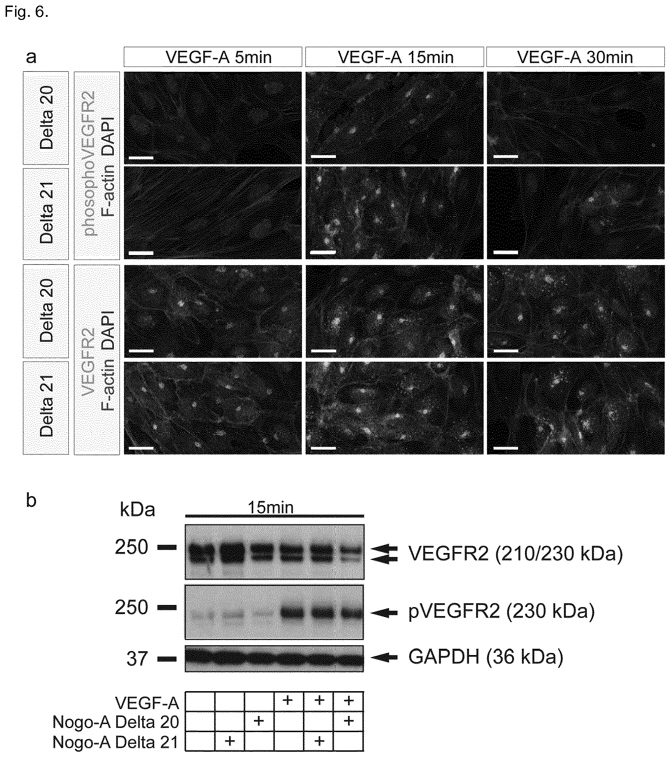

Fig. 6 shows that Nogo-A interacts with the VEGF-VEGFR pathway (which is of high clinical relevance, e.g. targeted by Bevacizumab/Avastin). (a) HUVECs were treated with VEGF-A for 5min, 15min, or 30min and Nogo-A Delta 20 or Nogo-A Delta 21. Phosphorylation of VEGR2 at Tyr1175 and total VEGFR2 was visualized with an immunofluorescent antibody staining. VEGFR2 phosphorylation peaked after 15min and was considerably lower in the Nogo-A Delta 20-treated conditions as compared to Nogo-A Delta 21 conditions. While in the Nogo-A Delta 21-treated conditions there was residual phosphoVEGFR2 after 30min, no phosphoVEGFR2 was detectable in the Nogo-A Delta 20 conditions. (b) HUVECs were treated with or without VEGF-A for 15min and Nogo-A Delta 20 or Nogo-A Delta 21. Phosphorylation of VEGFR2 at Tyr1175 and total VEGFR2 was visualized with western blot. Treatment with Nogo-A Delta 20 or Nogo-A Delta 21 alone did not induce VEGFR2 phosphorylation. Addition of VEGF-A strongly induced phosphorylation of VEGFR2 at Tyr1175. Addition of Nogo-A Delta 20 reduced the amount of phosphoVEGFR2 protein as well as the total amount of VEGFR2 protein. Scale bars: 20µm.

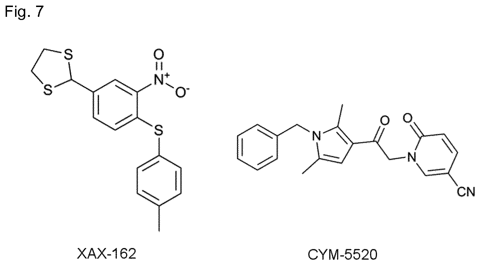

Fig 7. shows the chemical structure of S1PR2 agonists XAX-162 (left side) and CYM-5520 (right side) as described in Satsu et al., Bioorg Med Chem 2013;21, 5373-5382.

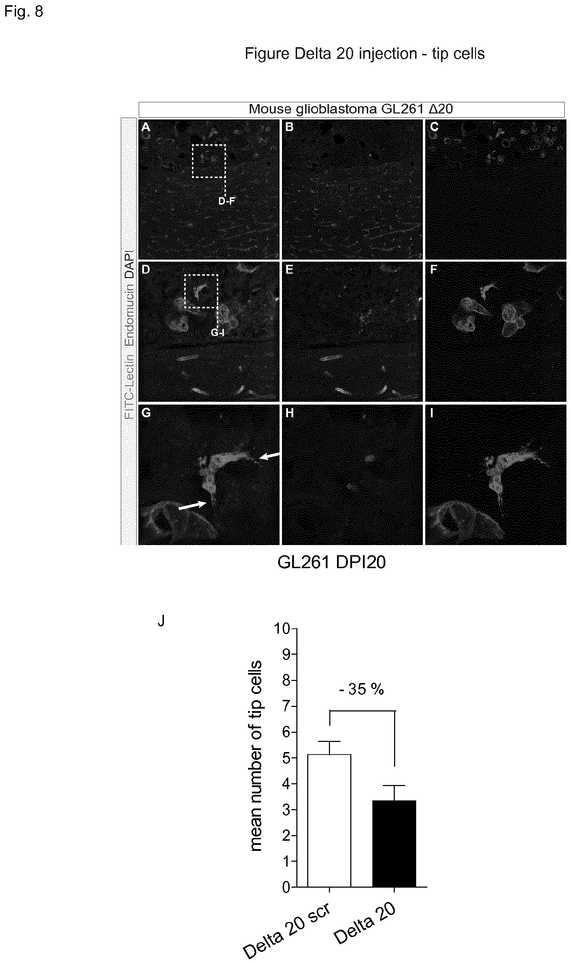

Fig. 8 shows that injection of Nogo-A Delta 20 inhibits growing blood vessels and endothelial tip cells (a specialized cell type at the forefront of growing blood vessels) in a mouse model of glioblastoma. Nogo-A Delta 20 and scrambled Nogo-A Delta 20 (inactive form of Nogo-A Delta 20) were injected into glioblastoma-bearing mice using osmotic minipumps. Coronal brain sections (40 µm) of glioblastoma-bearing mice (20 days post-injection, 20D-PI) were perfused with FITC Lectin (green) to visualize perfused blood vessels and stained for Endomucin to visualize blood vessels including endothelial tip cells (red) and the general nuclear marker DAPI (blue). The boxed areas are enlarged in d-f and in g-i. (a-f). Endomucin⁺ endothelial tip cells (red, arrows) and FITC Lectin⁺ established blood vessel (green) (d-f) are shown within the glioblastoma. Endomucin seems to be specific for blood vessels within the glioblastoma (red) (as compared to Endomucin⁻ blood vessels in the surrounding normal brain; green). (d,f,g,i) Note the nicely visualized Endomucin⁺ endothelial tip cells including filopodial protrusions (red, arrows) (g) within the glioblastoma. (j) Injection of Nogo-A Delta 20 decreases the mean number of endothelial tip cells within the glioblastoma, showing its inhibitory effect on mouse glioblastoma blood vessels.

### Examples

### Concepts and Evidence behind the Invention

Whereas it is known in the art that Nogo-A Delta 20 but not Nogo-66 is involved in the regulation of angiogenesis during mouse brain development (Wälchli et al., PNAS 2013;110, E1943-52), the disclosed data shows - in contrast - that human glioblastoma-derived endothelial cells are responsive to Nogo-66 as well as Nogo-A Delta 20-induced retraction of lamellipodia and filopodia (Fig.4) and inhibition of migration (Fig.5). These data highlight an important role of Nogo-A in glioblastoma vascularization and growth. However, it is likely that Nogo-A also affects astrocytomas of WHO grades I-III as well as other brain tumors (e.g. meningiomas, hemangiopericytomas etc.).

This invention is based on the above mentioned finding that two inhibitory domains of Nogo-A - Nogo-A Delta 20 and Nogo-66 - are capable of inhibiting human glioblastoma blood vessel growth. Without wishing to be bound by theory, the inventors hypothesize that this effect is achieved by interaction with their receptors - S1PR (for Nogo-A Delta 20) and Nogo-66 receptor complex (for Nogo-66), respectively. S1PRs comprise a group of five G protein-coupled receptors, S1PR1, S1PR2, S1PR3, S1PR4 and S1PR5, mediating a wide variety of biological functions.

### Sequence listing:

SEQ ID NO 003 >Rat Nogo-66 (66aa; aa 1026 - 1091)
   RIYKGVIQAIQKSDEGHPFRAYLESEVAISEELVQKYSNSALGHVNCTIKELRRLFLVDDLVDSLK
SEQ ID NO 004 >Human Nogo-66 (66aa; aa 1055 - 1120)
   RIYKGVIQAIQKSDEGHPFRAYLESEVAISEELVQKYSNSALGHVNCTIKELRRLFLVDDLVDSLK

### Example 1: Expression of S1PR2 on blood vessels in a mouse model of glioblastoma.

### Method:

GL261 glioma cell implantation into adult (two months old) C57BL/6 animals was performed as previously described (Aulwurm et al., Int J Cancer 2006;118, 1728-1735; Biollaz et al., Eur J Immunol 2009;39, 1323-1333j). Briefly, a total of 2×10⁴ GL261 cells were inoculated through a borehole placed 1mm lateral and 2mm dorsal to the bregma at 3mm depth using a Hamilton syringe. Tumor-bearing mice were checked daily by weight measurement and assessment of neurological symptoms associated with an intracranial tumor. At post-injection day 7 (7D-PI), 15 (15D-PI) and endpoint (3 weeks), animals were processed for immunofluorescence. In brief, mice were anaesthesized with isoflurane and i.v. injected with 150ug FITC-Lectin (Vector Laboratories) in 0.9% NaCl. FITC-Lectin was allowed to circulate for 5min before mice were intracardially perfused with 4% PFA. Subsequently, brains were dissected, postfixed in 4% PFA overnight at 4°C. Fixed tissue was cryoprotected in 30% sucrose in 0.1 M PBS for 3-4 days and then embedded in O.C.T. Tissue-Tec (Sakura). Tumor-bearing brains were cryosected into sections with a thickness of 40 µm. immunohistochemistry was performed as described (Wälchli et al., PNAS 2013;110, E1943-52).

### Results:

In Figure 1 the expression of S1PR2 in a mouse model of glioblastoma is shown. Blood vessels (Fig. 1a-c; green, arrows) in the normal brain adjacent to the glioblastoma express S1PR2 (Fig. 1 a-c; red). Within the tumor/glioblastoma/glioma, S1PR2 on blood vessels (Fig. 1 d-f; green, arrows) is upregulated.

### Example 2: Blood vessels express S1PR2 in a human glioblastoma.

### Method:

Tumor tissue from patients operated at the Division of Neurosurgery, University Hospital Zurich (Zurich, Switzerland) was fixed in 4% PFA overnight at 4°C. Fixed tissue was cryoprotected in 30% sucrose in 0.1M PBS for 3-4 days and then embedded in O.C.T. Tissue-Tec (Sakura). Tumor tissue was cryosected into sections with a thickness of 80 µm. Immunohistochemistry was performed as described (Wälchli et al., PNAS 2013;110, E1943-52).

### Results:

In Figure 2 expression of Nogo-A and S1PR2 in human glioblastoma tissue is demonstrated. Blood vessels (Fig. 2 a-c; red) within the human glioblastoma tissue express S1PR2 (Fig. 2 a-c; green). S1PR2 is expressed on an established blood vessel (Fig. 2 d-i; red) (d-f) as well as on endothelial tip cells (Fig. 2d-f; red, arrows) and within the glioblastoma (Fig. 2g-i).

Tissue microarrays (10 µm) of human glioblastoma and normal human brain samples (Fig. 2 j-q) were stained for Nogo-A (red) and for S1PR2 (brown). S1PR2 (brown) is expressed on endothelial cells of normal human brain as well as on tumor endothelial cells of human glioblastoma tissues (arrows). Nogo-A (red) shows a highly variable expression in human glioblastoma ranging from virtually absent (n,o) to strong expression (p,q). Nogo-A expression on tumor endothelial cells was almost never observed (n-q).

### Example 3 Nogo-A receptors are expressed in human glioblastoma and in human glioblastoma endothelial cells

### Method

Western blot and quantitative PCR was performed as describe previously (Wälchli et al., PNAS 2013;110, E1943-52).

### Results:

Western blots using antibodies against the different proteins of the receptors for Nogo-A Delta 20 and for Nogo-66. S1P receptors (S1PR1,2,3,5) were detected in human glioblastoma extracts (Glioblastoma ZH-379/413) and in isolated human glibolastoma endothelial cells (Glioblastoma ZH-379/413 MVEC) (Fig. 3 a). Protein members of the Nogo-66 receptor complex, namely NgR1, Lingo1, TROY, p75 and PirB were detected in human glioblastoma extracts (Glioblastoma ZH-379/413 and in isolated human glibolastoma endothelial cells (Glioblastoma ZH-379/413 MVEC).

Expression of S1PR2, S1PR1, and S1PR5 were also detected in normal brain MVEC and glioblastoma MVEC (GBM MVEC) using quantitative RT-PCR (Fig. 3 b). The expression was comparable to HUVEC and HUAEC except for S1PR1, which was stronger in HUVEC and HUAEC.

### Example 4: Nogo-A Delta 20 interacts with S1PR2 to mediate lamellipodia and filopodia retraction.

### Method:

For time-lapse- and force generation experiments, Vybrant/Dil (Invitrogen, 1:200)-labeled GBM MVEC (10⁴ cells ml⁻¹) were added onto the nanopillar structures. The imaging process was started 1h after seeding GBM MVEC on nanopillars. To calculate the traction force exerted by GBM MVEC on the nanopillar surface, the deflection of the nanopillar tip was imaged with confocal microscopy using a Leica confocal microscope SP5 with a63x, oil immersion, NA1.43 objective.

During image acquisition, GBM MVEC were kept at 37°C and 10% CO₂. Following laser wavelengths were used to acquire images of nanopillar arrays and Di-labeled cells: 488 nm (nanopillar structures) and 546 nm (Dil).

To quantitatively address the effects of coated Nogo-A proteins on adhesion between endothelial cells (GBM MVEC) and nanopillars, nanopillars were coated with Nogo-A Delta 20, Nogo-A Delta 21, or Fibronectin (blank condition).

To calculate the total force applied by endothelial cells (GBM MVEC) on the substrates, the average displacement of 800-5000 nanopillars per single cell was used. Single GBM MVEC were imaged for a total time of 20 min, with a scanning ratio of 30 s per frame. For each condition, at least three individual GBM MVEC were analyzed.

To address the role of Nogo-A receptors, endothelial cells were pre-incubated for 30min before seeding with the pharmacological blocker of S1PR2 (JTE-013, 10 µM) (Tocris Bioscience), with the pharmacological blocker of S1PR1 and S1PR3 (VPC 23019, 10 µM) (Tocris Bioscience), or with the pharmacological blocker of S1PR1, 3, 4, and 5 (FTY720, 10 µM) (Tocris Bioscience).

To quantitatively address the acute effects of soluble Nogo-A proteins on GBM MVEC retraction, Dil-labeled endothelial cells on nanopillars coated with 25 µg^{∗}ml⁻¹ human Fibronectin were treated 30min after cell seeding with soluble Nogo-A Delta 20 and Nogo-A Delta 21 at 1 µM. Single GBM MVEC were then imaged for a total time of 1.5 h, with a scanning ratio of 30 s per frame. The nanopillar displacements were recorded as sequence images when the GBM MVEC applied forces on the nanopillar surface. For each condition, at least three individual GBM MVEC were analyzed. To address the role of Nogo-A receptors, endothelial cells were cultured for 30min before incubating the cells for another 30min with the pharmacological blocker of S1PR2 (JTE-013, 10 µM) (Tocris Bioscience), with the pharmacological blocker of S1PR1 and S1PR3 (VPC 23019, 10 µM) (Tocris Bioscience), or with the pharmacological blocker of S1PR1, 3, 4, and 5 (FTY720, 10 µM) (Tocris Bioscience). Then soluble Nogo-A Delta 20 (at 1 µM) or Nogo-A Delta 21 (at 1 µM) was added and lamellipodia and filopodia were imaged for 1h. The displacements (x) of the nanopillar tips induced by whole endothelial cells (for experiments with coated Nogo-A proteins) or by single endothelial filopodia (for experiments with soluble Nogo-A proteins) were measured using the program Diatrack (Powerful Particle Tracking, Semasopht). Finally, corresponding traction forces were calculated using the formula F=k^{∗}x, where k represents the spring constant of the nanopillars, 78.76 ηN/µm.

### Results

Time course showing the retraction of isolated human glioblastoma microvascular endothelial cell (GBM MVECs) lamellipodia and filopodia over 30 min to 1 h, induced by Nogo-A Delta 20 at a final concentration of 1 • M (Fig. 4a-d). Nogo-A Delta 21-treated GB-MVEC showed only minor shape changes (Fig. 4 e-h). Soluble Nogo-A Delta 20 (1 • M)-induced retraction of GBM MVEC lamellipodia and filopodia was counterbalanced by pre-incubation of GBM MVECs with JTE-013 (10 • M), a pharmacological inhibitor of S1PR2 (Fig. 4 i-l).

Mean traction forces and pillar displacements induced by adhered GB-MVECs on nanopillars were reduced on Nogo-A Delta 20-coated nanopillars but not on Nogo-A Delta 21-coated nanopillars (Fig. 4m). Nogo-A Delta 20 coating induced mean traction forces on the underlying nanopillars of ∼12 nN. Pre-incubation of GBM MVECs with 10 • M of the pharmacological S1PR2 inhibitor JTE-013 decreased the mean traction forces induced by Nogo-A Delta 20 coating onto the nanopillars to ∼4.1 nN. However, pre-incubation of GBM MVECs with 10 • M of the pharmacological inhibitor of S1PR1 and S1PR3, VPC 23019 or with the pharmacological inhibitor of S1PR1,3,4, or 5, FTY720 did not decrease the mean traction forces induced by Nogo-A Delta 20 coating onto the nanopillars,
Peak traction forces exerted by GBM MVEC filopodia onto nanopillars. Nogo-A Delta 20 (1 • M) treated GBM MVEC filopodia induced peak traction forces on the underlying nanopillars of -10 nN, while treatment with the control peptide Nogo-A Delta 21 induced peak traction forces of only ∼4 nN (Fig. 4n). Pre-incubation of GBM MVECs with 10 • M of the pharmacological S1PR2 inhibitor JTE-013 decreased the average peak traction forces induced by Nogo-A Delta 20 (1 • M) treated GBM MVEC filopodia onto the nanopillars to ∼4.5 nN, almost down to the levels of Nogo-A Delta 21 (1 • M)-treated GBM MVEC filopodia (∼4 nN). Interestingly, pre-incubation of GBM MVECs with 10 • M of the pharmacological inhibitor of S1PR1 and S1PR3, VPC 29013 or with the pharmacological inhibitor of S1PR1,3,4, or 5, FTY720 did also decrease the average peak traction forces induced by Nogo-A Delta 20 (1 • M) treated GBM MVEC filopodia onto the nanopillars, to levels close to Nogo-A Delta 21.

### Example 5: Nogo-A Delta 20 and Nogo-66 inhibit GBM MVEC migration via S1PR2 and via NgR1, respectively

### Method:

For the migration assays, the undersides of 24-well plate inserts (FluoroBlock, BD Falcon) were coated with 0.5 ml PBS containing 10 µg^{∗}ml⁻¹ fibronectin (recombinant human fibronectin, Biopur AG) and different concentrations of Nogo-A Delta 21, Nogo-A Delta 20 and Nogo-66 (R&D Systems) and incubated for 1 h at 37°C. Endothelial cells (GBM MVECs) were starved in migration medium (DMEM + 0.25% BSA) 4 h before detachment with accutase (PAA Laboratories). Cells were resuspended in migration medium (1 Mio ml⁻¹) and labeled with calcein AM (Molecular Probes, 4 µg ml⁻¹), followed by washing with HBSS and resuspending in migration medium (125,000 cells ml⁻¹). The coated inserts were washed and the bottom chamber was prepared by adding migration medium with or without human VEGF-A (PeproTech Inc., 10 ng ml⁻¹). The inserts were then put in an empty 24-well plate (BD Falcon) and calcein AM-labeled endothelial cells (50,000 cells per insert) were added.

The loaded inserts were transferred to the prepared cluster plate and incubated at 37°C, 5% CO₂ for 4 h. A cell titration plate was prepared by adding different cell numbers (50,000/25,000/12,500/6,250/3,125/1,563/782 cells per well) in triplicates in 1.2 ml migration medium and incubated in parallel. The fluorescence was measured after 4 h by a cytofluorometer (Tean Infinite Pro 200) and the number of migrated cells was calculated based on the titration curve.

To ask how co-signaling of Nogo-A Delta 20/Nogo-66 coated to the underside of transwell inserts and of its receptors affect cell migration, calcein-AM labeled GBM MVECs were incubated for 1h with the pharmacological blockers of-the Nogo-A Delta 20 receptor S1PR2 (JTE-013, 1 µM, 10 µM), with the pharmacological blocker of S1PR1 and S1PR3 (VPC 23019, 10 µM) (Tocris Bioscience), and with the competitive inhibitor of the Nogo-66 receptor NgR1 (NEP1-40, 4.6 µg/ml) during calcein-AM labeling. After washing, the cell medium was again supplied with these pharmacological blockers, prior to cell addition in the upper chamber of the transwell insert.

### Results:

Human GBM MVECs show induced migration upon stimulation with a combination of VEGF-A and Fibronectin (VEGF+FN) (schematic in (Fig. 5a) and data in (Fig. 5b)). VEGF+FN-induced migration of human GBM MVECs is dose-dependently inhibited by the addition of Nogo-A Delta 20 (Fig.5c). Pre-incubation of GBM MVECs with 10 • M of the pharmacological S1PR2 inhibitor JTE-013 significantly increases the migration potential despite Nogo-A Delta 20 exposure (Fig. 5d). Although VPC 23019, a pharmacological blocker for S1PR1 and S1PR3, has no effect on its own, combined pre-incubation with JTE-013 lead to a strong reduction of the inhibitor effect of Nogo-A Delta 20 on GBM MVEC migration. VEGF+FN-induced migration of human GBM MVECs is dose-dependently inhibited by the addition of Nogo-66 (Fig. 5e). Pre-incubation with NEP1-40, a competitive antagonist of Nogo-66, significantly increases the migration potential GBM MVEC upon exposure to Nogo-66 coating.

### Example 6: Nogo-A Delta 20 interacts with the VEGF-VEGFR2 pathway to modulate signalling output

### Method:

For the different treatment conditions HUVECs were seeded on 8 well dishes (LabTek chamber slides, immunofluorescence) or 6 well dishes (western blot) and left for 2 to 3 days until the culture was confluent. After a 4h starvation of the cells in EBM-2 medium (Lonza, Switzerland), the cells were treated with Nogo-A Delta 20 (1µM), Nogo-A Delta 20 (1µM) and VEGF-A (100ng/µl), Nogo-A Delta 21 (1µM), Nogo-A Delta 21 (1µM) and VEGF (100ng/µl), or VEGF-A alone (100ng/µl) in EBM-2 medium, respectively. The cells were either fixed in 4% formaldehyde for 15min at room temperature or lysed in RIPA buffer. After fixation, HUVECs were stained with anti-phosphoVEGFR2 antibody (pTyr1175, #2478, Cell Signaling), F-actin (phalloidin Alexa Fluor 633, Molecular Probes) and DAPI. The same antibodies were used to detect VEGFR2 and phosphoVEGFR2 in western blot.

### Results:

HUVECs were treated with VEGF-A for 5min, 15min, or 30min and Nogo-A Delta 20 or Nogo-A Delta 21 (Fig. 6a). Phosphorylation of VEGFR2 at Tyr1175 and total VEGFR2 was visualized with an immunofluorescent antibody staining. VEGFR2 phosphorylation peaked after 15min and was considerably lower in the Nogo-A Delta 20-treated conditions as compared to Nogo-A Delta 21 conditions. While in the Nogo-A Delta 21-treated conditions there was residual phosphoVEGFR2 after 30min, no phosphoVEGFR2 was detectable in the Nogo-A Delta 20 conditions
HUVECs were treated with or without VEGF-A for 15min and Nogo-A Delta 20 or Nogo-A Delta 21 (Fig. 6b). Phosphorylation of VEGFR2 at Tyr1175 and total VEGFR2 was visualized with western blot. Treatment with Nogo-A Delta 20 or Nogo-A Delta 21 alone did not induce VEGFR2 phosphorylation. Addition of VEGF-A strongly induced phosphorylation of VEGFR2 at Tyr1175. Addition of Nogo-A Delta 20 reduced the amount of phosphoVEGFR2 protein as well as the total amount of VEGFR2 protein.

## Claims

1. An extracellular domain of Nogo selected from Nogo-A delta 20, for use in a method for the treatment of glioblastoma.

2. The extracellular domain of Nogo for use in a method for the treatment of glioblastoma according to claim 1, wherein the extracellular domain has > 70%, ≥ 80%, ≥ 85%, ≥ 90%, ≥ 92%, ≥ 93%, ≥ 94%, ≥ 95%, ≥ 96%, ≥ 97% or ≥ 98% sequence identity compared to any one of SEQ ID NO 001 or SEQ ID NO 002.

3. A pharmaceutical composition for use in a method for the treatment of glioblastoma, said pharmaceutical composition comprising a polypeptide comprising an extracellular domain of Nogo selected from Nogo-A delta 20.

4. A pharmaceutical composition for use in a method for the treatment of glioblastoma according to claim 3, wherein the extracellular domain has ≥ 70%, ≥ 80%, ≥ 85%, ≥ 90%, ≥ 92%, ≥ 93%, ≥ 94%, ≥ 95%, ≥ 96%, ≥ 97% or ≥ 98% sequence identity compared to any one of SEQ ID NO 001 or SEQ ID NO 002.

5. The pharmaceutical composition for use according to claim 3 or 4, wherein the polypeptide comprises Nogo-A delta 20.

6. The pharmaceutical composition for use according to any one of claims 3 to 5 for use in a method for the treatment of glioblastoma by intravenous or intrathecal administration.

7. An extracellular domain of Nogo selected from Nogo-66, for use in a method for the treatment of glioblastoma by inhibition of glioblastoma angiogenesis.

8. The extracellular domain of Nogo for use in a method for the treatment of glioblastoma by inhibition of glioblastoma angiogenesis according to claim 7, wherein the extracellular domain has ≥ 70%, ≥ 80%, ≥ 85%, ≥ 90%, ≥ 92%, ≥ 93%, ≥ 94%, ≥ 95%, ≥ 96%, ≥ 97% or ≥ 98% sequence identity compared to any one of SEQ ID NO 003 or SEQ ID NO 004.

## Patentansprüche

1. Extrazelluläre Domäne von Nogo, ausgewählt aus Nogo-A Delta 20, zur Verwendung in einem Verfahren zur Behandlung des Glioblastoms.

2. Extrazelluläre Domäne von Nogo zur Verwendung in einem Verfahren zur Behandlung des Glioblastoms nach Anspruch 1, wobei die extrazelluläre Domäne ≥ 70 %, ≥ 80 %, ≥ 85 %, ≥ 90 %, ≥ 92 %, ≥ 93 %, ≥ 94 %, ≥ 95 %, ≥ 96 %, ≥ 97 % oder ≥ 98 % Sequenzidentität im Vergleich mit einer von SEQ ID NO 001 oder SEQ ID NO 002 aufweist.

3. Pharmazeutische Zusammensetzung zur Verwendung in einem Verfahren zur Behandlung des Glioblastoms, wobei die pharmazeutische Zusammensetzung ein Polypeptid umfasst, das eine extrazelluläre Domäne von Nogo umfasst, ausgewählt aus Nogo-A Delta 20.

4. Pharmazeutische Zusammensetzung zur Verwendung in einem Verfahren zur Behandlung des Glioblastoms nach Anspruch 3, wobei die extrazelluläre Domäne ≥ 70 %, ≥ 80 %, ≥ 85 %, ≥ 90 %, ≥ 92 %, ≥ 93 %, ≥ 94 %, ≥ 95 %, ≥ 96 %, ≥ 97 % oder ≥ 98 % Sequenzidentität im Vergleich mit einer von SEQ ID NO 001 oder SEQ ID NO 002 aufweist.

5. Pharmazeutische Zusammensetzung zur Verwendung nach Anspruch 3 oder 4, wobei das Polypeptid Nogo-A Delta 20 umfasst.

6. Pharmazeutische Zusammensetzung zur Verwendung nach einem der Ansprüche 3 bis 5 zur Verwendung in einem Verfahren zur Behandlung des Glioblastoms durch intravenöse oder intrathekale Verabreichung.

7. Extrazelluläre Domäne von Nogo, ausgewählt aus Nogo-66, zur Verwendung in einem Verfahren zur Behandlung des Glioblastoms durch Hemmung der Angiogenese des Glioblastoms.

8. Extrazelluläre Domäne von Nogo zur Verwendung in einem Verfahren zur Behandlung des Glioblastoms durch Hemmung der Angiogenese des Glioblastoms nach Anspruch 7, wobei die extrazelluläre Domäne ≥ 70 %, ≥ 80 %, ≥ 85 %, ≥ 90 %, ≥ 92 %, ≥ 93 %, ≥ 94 %, ≥ 95 %, ≥ 96 %, ≥ 97 % oder ≥ 98 % Sequenzidentität im Vergleich mit einer von SEQ ID NO 003 oder SEQ ID NO 004 aufweist.

## Revendications

1. Domaine extracellulaire de Nogo choisi parmi Nogo-A delta 20, pour l'utilisation dans une méthode pour le traitement du glioblastome.

2. Domaine extracellulaire de Nogo pour l'utilisation dans une méthode pour le traitement du glioblastome selon la revendication 1, dans lequel le domaine extracellulaire a ≥ 70 %, ≥ 80 %, ≥ 85 %, ≥ 90 %, ≥ 92 %, ≥ 93 %, ≥ 94 %, ≥ 95 %, ≥ 96 %, ≥ 97 % ou ≥ 98 % d'identité de séquence par comparaison avec l'une quelconque de SEQ ID NO : 001 ou SEQ ID NO : 002.

3. Composition pharmaceutique pour l'utilisation dans une méthode pour le traitement du glioblastome, ladite composition pharmaceutique comprenant un polypeptide comprenant un domaine extracellulaire de Nogo choisi parmi Nogo-A delta 20.

4. Composition pharmaceutique pour l'utilisation dans une méthode pour le traitement de glioblastome selon la revendication 3, dans laquelle le domaine extracellulaire a ≥ 70 %, ≥ 80 %, ≥ 85 %, ≥ 90 %, ≥ 92 %, ≥ 93 %, ≥ 94 %, ≥ 95 %, ≥ 96 %, ≥ 97 % ou ≥ 98 % d'identité de séquence par comparaison avec l'une quelconque de SEQ ID NO : 001 ou SEQ ID NO : 002.

5. Composition pharmaceutique pour l'utilisation selon l'une des revendications 3 ou 4, dans laquelle le polypeptide comprend Nogo-A delta 20.

6. Composition pharmaceutique pour l'utilisation selon l'une quelconque des revendications 3 à 5 pour une utilisation dans une méthode pour le traitement du glioblastome par administration intraveineuse ou intrathécale.

7. Domaine extracellulaire de Nogo choisi parmi Nogo-66, pour l'utilisation dans une méthode pour le traitement du glioblastome par inhibition de l'angiogenèse du glioblastome.

8. Domaine extracellulaire de Nogo pour l'utilisation dans une méthode pour le traitement du glioblastome par inhibition de l'angiogenèse du glioblastome selon la revendication 7, dans lequel le domaine extracellulaire a ≥ 70 %, ≥ 80 %, ≥ 85 %, ≥ 90 %, ≥ 92 %, ≥ 93 %, ≥ 94 %, ≥ 95 %, ≥ 96 %, ≥ 97 % ou ≥ 98 % d'identité de séquence par comparaison avec l'une quelconque de SEQ ID NO : 003 ou SEQ ID NO : 004.


### SEQUENCE LISTING

<110> Universitaet Zuerich
<120> Agonists of the Nogo-A S1PR pathway for the treatment of glioblastoma
<130> uz238wo
<160> 4
<170> PatentIn version 3.5
<210> 1
   <211> 182
   <212> PRT
   <213> rattus norvegicus
<400> 1
   Thr Gly Thr Lys Ile Ala Tyr Glu Thr Lys Val Asp Leu Val Gln Thr
   1               5                   10                  15
   Ser Glu Ala Ile Gln Glu Ser Leu Tyr Pro Thr Ala Gln Leu Cys Pro
               20                  25                  30
   Ser Phe Glu Glu Ala Glu Ala Thr Pro Ser Pro Val Leu Pro Asp Ile
           35                  40                  45
   Val Met Glu Ala Pro Leu Asn Ser Leu Leu Pro Ser Ala Gly Ala Ser
       50                  55                  60
   Val Val Gln Pro Ser Val Ser Pro Leu Glu Ala Pro Pro Pro Leu Ser
   65                  70                  75                  80
   Tyr Asp Ser Ile Lys Leu Glu Pro Glu Asn Pro Pro Pro Tyr Glu Glu
                   85                  90                  95
   Ala Met Asn Val Ala Leu Lys Ala Leu Gly Thr Lys Glu Gly Ile Lys
               100                 105                 110
   Glu Pro Glu Ser Phe Asn Ala Ala Val Gln Glu Thr Glu Ala Pro Tyr
           115                 120                 125
   Ile Ser Ile Ala Cys Asp Leu Ile Lys Glu Thr Lys Leu Ser Thr Glu
       130                 135                 140
   Pro Ser Pro Asp Phe Ser Asn Tyr Ser Glu Ile Ala Lys Phe Glu Lys
   145                 150                 155                 160
   Ser Val Pro Glu His Ala Glu Leu Val Glu Asp Ser Ser Pro Glu Ser
                   165                 170                 175
   Glu Pro Val Asp Leu Phe
               180
<210> 2
   <211> 182
   <212> PRT
   <213> homo sapiens
<400> 2
   Thr Gly Thr Lys Ile Ala Tyr Glu Thr Lys Met Asp Leu Val Gln Thr
   1               5                   10                  15
   Ser Glu Val Met Gln Glu Ser Leu Tyr Pro Ala Ala Gln Leu Cys Pro
               20                  25                  30
   Ser Phe Glu Glu Ser Glu Ala Thr Pro Ser Pro Val Leu Pro Asp Ile
           35                  40                  45
   Val Met Glu Ala Pro Leu Asn Ser Ala Val Pro Ser Ala Gly Ala Ser
       50                  55                  60
   Val Ile Gln Pro Ser Ser Ser Pro Leu Glu Ala Ser Ser Val Asn Tyr
   65                  70                  75                  80
   Glu Ser Ile Lys His Glu Pro Glu Asn Pro Pro Pro Tyr Glu Glu Ala
                   85                  90                  95
   Met Ser Val Ser Leu Lys Lys Val Ser Gly Ile Lys Glu Glu Ile Lys
               100                 105                 110
   Glu Pro Glu Asn Ile Asn Ala Ala Leu Gln Glu Thr Glu Ala Pro Tyr
           115                 120                 125
   Ile Ser Ile Ala Cys Asp Leu Ile Lys Glu Thr Lys Leu Ser Ala Glu
       130                 135                 140
   Pro Ala Pro Asp Phe Ser Asp Tyr Ser Glu Met Ala Lys Val Glu Gln
   145                 150                 155                 160
   Pro Val Pro Asp His Ser Glu Leu Val Glu Asp Ser Ser Pro Asp Ser
                   165                 170                 175
   Glu Pro Val Asp Leu Phe
               180
<210> 3
   <211> 66
   <212> PRT
   <213> rattus norvegicus
<400> 3
   Arg Ile Tyr Lys Gly Val Ile Gln Ala Ile Gln Lys Ser Asp Glu Gly
   1               5                   10                  15
   His Pro Phe Arg Ala Tyr Leu Glu Ser Glu Val Ala Ile Ser Glu Glu
               20                  25                  30
   Leu Val Gln Lys Tyr Ser Asn Ser Ala Leu Gly His Val Asn Cys Thr
           35                  40                  45
   Ile Lys Glu Leu Arg Arg Leu Phe Leu Val Asp Asp Leu Val Asp Ser
       50                  55                  60
   Leu Lys
   65
<210> 4
   <211> 66
   <212> PRT
   <213> homo sapiens
<400> 4
   Arg Ile Tyr Lys Gly Val Ile Gln Ala Ile Gln Lys Ser Asp Glu Gly
   1               5                   10                  15
   His Pro Phe Arg Ala Tyr Leu Glu Ser Glu Val Ala Ile Ser Glu Glu
               20                  25                  30
   Leu Val Gln Lys Tyr Ser Asn Ser Ala Leu Gly His Val Asn Cys Thr
           35                  40                  45
   Ile Lys Glu Leu Arg Arg Leu Phe Leu Val Asp Asp Leu Val Asp Ser
       50                  55                  60
   Leu Lys
   65